# EUROPÄISCHE PATENTSCHRIFT

(11) **EP 3 077 469 B1**
(45) Veröffentlichungstag und Bekanntmachung des Hinweises auf die Patenterteilung: **04.10.2017**
(21) Anmeldenummer: 14798903.2
(22) Anmeldetag: 17.11.2014
(51) Int. Cl.: C09J 7/02, B32B 7/06

(54) **VORLÄUFERVERBUNDMATERIAL, VERBUNDMATERIAL, VERFAHREN ZUR HERSTELLUNG EINES VORLÄUFERVERBUNDMATERIALS, VERFAHREN ZUR HERSTELLUNG EINES VERBUNDMATERIALS UND VERWENDUNG EINES VORLÄUFERVERBUNDMATERIALS UND EINES VERBUNDMATERIALS**
PRECURSOR COMPOSITE MATERIAL, COMPOSITE MATERIAL, METHOD FOR PRODUCING A PRECURSOR COMPOSITE MATERIAL, METHOD FOR PRODUCING A COMPOSITE MATERIAL AND USE OF A PRECURSOR COMPOSITE MATERIAL AND OF A COMPOSITE MATERIAL
MATÉRIAU COMPOSITE PRÉCURSEUR, MATÉRIAU COMPOSITE, PROCÉDÉ DE FABRICATION D'UN MATÉRIAU COMPOSITE PRÉCURSEUR, PROCÉDÉ DE FABRICATION D'UN MATÉRIAU COMPOSITE, ET UTILISATION D'UN MATÉRIAU COMPOSITE PRÉCURSEUR ET D'UN MATÉRIAU COMPOSITE

(30) Priorität: 05.12.2013 DE 102013113532
(43) Veröffentlichungstag der Anmeldung: 12.10.2016
(73) Patentinhaber: Infiana Germany GmbH & Co. KG, 91301 Forchheim (DE)
(72) Erfinder: SCHUHMANN, Michael, 90613 Grosshabersdorf (DE); HERMANN, Christian, 91330 Eggolsheim (DE)
(74) Vertreter: Epping - Hermann - Fischer
(86) Internationale Anmeldenummer: PCT/EP2014/074771
(87) Internationale Veröffentlichungsnummer: WO 2015/082200

(56) Entgegenhaltungen:
- EP-A2- 1 113 059
- DE-T2- 69 824 274
- GB-A- 2 068 833

## Beschreibung

Es wird ein Vorläuferverbundmaterial, ein Verbundmaterial, das aus dem Vorläuferverbundmaterial gebildet ist, ein Verfahren zur Herstellung des Vorläuferverbundmaterials, ein Verfahren zur Herstellung des Verbundmaterials sowie die Verwendung des Vorläuferverbundmaterials und des Verbundmaterials angegeben.

Verbundmaterialien, wie sie beispielsweise in Hygienelaminaten oder für Labels eingesetzt werden, haben bislang den Nachteil, dass sie, um eine gewisse Festigkeit beizubehalten, eine für viele Anwendungen zu große Dicke haben. Dies kann beispielsweise bei Hygienelaminaten, wie Damenbinden oder Slipeinlagen, zu eingeschränktem Tragekomfort führen.

Die **Druckschrift** GB 2068833 A offenbart eine Laminatkonstruktion mit einem Oberflächenmaterialbogen, eine Polymermaterialschicht, eine Klebeschicht in Kontakt mit einem "Release-Liner" oder einem Substrat. Zwischen dem Oberflächenmaterialbogen und der Polymermaterialschicht können zudem Mittel zur Abtrennung des Oberflächenmaterialbogens von der Polymermaterialschicht vorliegen, wie den Figuren la bis 1d und Seite 2, Z. 14 bis S. 3, Z. 35 dieser Druckschrift zu entnehmen ist. Die Schichtenfolge wird in Figur 1b veranschaulicht. Die in diesem Dokument beschriebene Klebeschicht befindet sich im Inneren der dort vorgesehenen Schichtenanordnung. Weiterhin steht die in diesem Dokument beschriebene Klebeschicht in Kontakt mit einem "Release-Liner" oder einem Substrat und wird von selbigen bedeckt.

Die **Druckschrift** DE 69824274 T2 offenbart ein Klebeband zum lösbaren Ankleben einer Einlage z.B. einer Zeitung (Absatz [0001] dieser Druckschrift). Ein derartiges Klebeband, wie in Figur 4 dieser Druckschrift gezeigt, weist einen Release-Liner, eine erste Klebeschicht und eine zweite Klebeschicht, einen Träger sowie eine nicht klebende Polymerschicht und eine Ablöseregulierungsschicht auf.

Aufgabe zumindest einer Ausführungsform der Erfindung ist die Bereitstellung eines Vorläuferverbundmaterials, aus dem ein Verbundmaterial mit verbesserten Eigenschaften gebildet werden kann, sowie die Bereitstellung eines solchen Verbundmaterials mit verbesserten Eigenschaften. Weitere Aufgaben weiterer Ausführungsformen sind die Bereitstellung von Verfahren zur Herstellung eines Vorläuferverbundmaterials und eines Verbundmaterials mit verbesserten Eigenschaften sowie die Verwendung des Vorläuferverbundmaterials zur Herstellung des Verbundmaterials und die Verwendung des Verbundmaterials.

Es wird ein Vorläuferverbundmaterial angegeben, das eine Schichtenfolge aufweist, die eine Klebeschicht, eine Trägerschicht auf der Klebeschicht, eine Releaseschicht auf der Trägerschicht und eine Trennschicht auf der Releaseschicht enthält, wobei die Schichtenfolge so angeordnet ist, dass die Klebeschicht mit ihrer von der Schichtenfolge abgewandten Seite zumindest in Teilbereichen auf der von der Schichtenfolge abgewandten Seite der Trennschicht angeordnet ist und wobei zwischen der Klebeschicht und der Trennschicht eine Klebekraft vorhanden ist, die größer ist als eine Delaminationskraft zwischen der Trägerschicht und der Releaseschicht.

Unter "auf" in Bezug auf die Anordnung der Schichten der Schichtenfolge ist eine Anordnung der einzelnen Schichten zu verstehen, bei der die einzelnen Schichten gemeinsame Grenzflächen miteinander aufweisen. Die einzelnen Schichten der Schichtenfolge, also Klebeschicht, Trägerschicht, Releaseschicht und Trennschicht, können jedoch jeweils Teilschichten aufweisen, die unterschiedliche Materialien enthalten. Beispielsweise kann die Trägerschicht eine erste Trägerschicht und eine zweite Trägerschicht aufweisen und die Releaseschicht eine erste Releaseschicht, eine zweite Releaseschicht und eine dritte Releaseschicht, und beispielsweise die erste Releaseschicht auf der zweiten Trägerschicht angeordnet sein, also eine gemeinsame Grenzfläche mit ihr aufweisen.

Unter "auf" in Bezug auf die Anordnung der Schichtenfolge ist ebenfalls eine direkte Anordnung zu verstehen, also eine gemeinsame Grenzfläche der von der Schichtenfolge abgewandten Seite der Klebeschicht und der von der Schichtenfolge abgewandten Seite der Trennschicht zumindest in Teilbereichen der Schichtenfolge.

Das Vorläuferverbundmaterial enthält somit eine Schichtenfolge, die einen Verbund von Schichten aufweist, und die durch ihre Anordnung selbst wieder einen Verbund von Schichtenfolgen bildet. Das bedeutet, dass bei einem Querschnitt durch das Vorläuferverbundmaterial mindestens zwei Schichtenfolgen übereinander angeordnet vorliegen.

Die Schichtenfolge eines solchen Vorläuferverbundmaterials weist eine höhere Zugfestigkeit auf als die einzelnen Schichten der Schichtenfolge des Vorläuferverbundmaterials. Daher kann das Vorläuferverbundmaterial weiter zu einem Verbundmaterial verarbeitet werden, ohne dass einzelne oder alle Schichten der Schichtenfolge in dem Verarbeitungsprozess zu Schaden kommen.

Weiterhin ist zwischen der Klebeschicht und der Trennschicht eine Klebekraft vorhanden, die größer ist als eine Delaminationskraft zwischen der Trägerschicht und der Releaseschicht. Unter "Delaminationskraft" ist hier und im Folgenden eine Lösekraft zu verstehen, die aufgebracht werden muss, um eine Delamination der Trägerschicht von der Releaseschicht zu bewirken. Dadurch kann, wenn die Schichtenfolge in ihrer Anordnung geändert werden soll, also beispielsweise eine gewickelte Schichtenfolge abgewickelt werden soll, erreicht werden, dass eine Delamination, also eine Trennung der Trägerschicht und der Releaseschicht erfolgt, während die Klebeschicht und die Trennschicht ihre gemeinsame Grenzfläche beibehalten.

Dadurch kann durch ein wie oben beschrieben angeordnetes Vorläuferverbundmaterial einfach ein Verbundmaterial mit einer von dem Vorläuferverbundmaterial unterschiedlichen Abfolge der Schichten erzeugt werden. Während das Vorläuferverbundmaterial mit der Schichtenfolge Klebeschicht-Trägerschicht-Releaseschicht-Trennschicht produziert wird, kann einfach durch Delamination der Trägerschicht und der Releaseschicht ein Verbundmaterial erhalten werden, welches eine Abfolge der Schichten Releaseschicht-Trennschicht-Klebeschicht-Trägerschicht aufweist. Insbesondere durch die Auswahl der Materialien der einzelnen Schichten des Vorläuferverbundmaterials wird somit ein kontrollierter Delaminationsprozess ermöglicht.

Dadurch kann durch einfaches Umsortieren der Schichtenfolge des Vorläuferverbundmaterials ohne Zugabe weiterer Materialien und/oder Entfernen von im Vorläuferverbundmaterial vorhandener Komponenten ein Verbundmaterial hergestellt werden. Abgesehen von der Abfolge der einzelnen Schichten in dem Verbundmaterial werden somit die Eigenschaften der einzelnen Schichten des Verbundmaterials bereits durch das Vorläuferverbundmaterial bestimmt.

In einer Ausführungsform weist das Vorläuferverbundmaterial eine Schichtenfolge auf, die gewickelt ist. Durch die Wickelung wird erreicht, dass die Klebeschicht mit ihrer von der Schichtenfolge abgewandten Seite zumindest in Teilbereichen auf der von der Schichtenfolge abgewandte Seite der Trennschicht angeordnet ist. Lediglich die äußerste Lage der Wickelung weist in einem solchen Fall einen Teilbereich der Trennschicht beziehungsweise der Klebeschicht auf, die frei von der jeweils anderen Schicht sind. Bei einer Wickelung der Schichtenfolge liegen im Inneren der Wickelung je nach Länge der Schichtenfolge mindestens zwei Schichtenfolgen übereinander vor.

Die Releaseschicht kann dabei eine Dicke aufweisen, die kleiner als 40 µm, bevorzugt kleiner als 30 µm, besonders bevorzugt kleiner als 20 µm, ganz besonders bevorzugt kleiner als 15 µm ist und/oder die Trägerschicht eine Dicke aufweisen, die kleiner als 20 µm, bevorzugt kleiner als 15 µm, besonders bevorzugt kleiner als 10 µm, ganz besonders bevorzugt kleiner 5 µm ist. Beispielsweise kann die Releaseschicht weniger als 100 gsm (Gramm pro m²), bevorzugt weniger als 50 gsm, besonders bevorzugt weniger als 20 gsm, ganz besonders bevorzugt weniger als 15 gsm dick sein. Damit können deutlich dünnere Release- und/oder Trägerschichten bereitgestellt werden als im Stand der Technik. Beispielsweise weisen herkömmliche Trägerschichten eine Dicke von 20 bis 30 µm auf und herkömmliche Releaseschichten eine Dicke von etwa 40 µm.

Damit enthält das Vorläuferverbundmaterial eine Releaseschicht und/oder eine Trägerschicht, welche besonders dünn ausgeformt sein können. Die besonders dünn ausgeformten Releaseschicht und/oder Trägerschicht sind bei der Herstellung des Vorläuferverbundmaterials sowie bei seiner Weiterverarbeitung zu einem Verbundmaterial jedoch nie alleine einem Zug, beispielsweise in einer Produktions- oder Veredelungsmaschine, unterzogen, sodass sie ohne Zerstörung produziert und verarbeitet werden können. Dies wäre ohne das Vorhandensein der zusätzlichen Schichten der Schichtenfolge des Vorläuferverbundmaterials nicht möglich.

Somit kann aus dem Vorläuferverbundmaterial ein Verbundmaterial hergestellt werden, welches besonders dünne Release- und/oder Trägerschichten aufweist, was wiederum zu vorteilhaften Eigenschaften beim Einsatz des Verbundmaterials führt. Auch bei der kontrollierten Delamination der Trägerschicht und der Releaseschicht steht demnach nicht die dünnste Schicht alleine unter Zug, sondern immer ein Laminat aus Schichten.

Weiterhin kann die Dicke der Klebeschicht kleiner als 50 gsm, bevorzugt kleiner als 30 gsm, bevorzugt kleiner als 15 gsm, ganz besonders bevorzugt kleiner als 5 gsm sein, beispielsweise 2 bis 5 gsm. Weiterhin kann die Trennschicht eine Dicke aufweisen, die kleiner als 10 gsm, bevorzugt kleiner als 5 gsm, besonders bevorzugt kleiner als 2 gsm ist, beispielsweise 0,5 bis 1 gsm.

Gemäß einer Ausführungsform weist das Vorläuferverbundmaterial eine Klebeschicht auf, welche ein Material aufweist, das aus einer Gruppe ausgewählt ist, die Haftklebstoffe umfasst. Unter "Haftklebstoff" soll hier und im Folgenden ein Klebstoff verstanden werden, der nach dem Auftragen auf die Trägerschicht dauerklebrig und/oder hochviskos bleibt. Der Haftklebstoff kann aus einer Gruppe ausgewählt sein, die lösungsmittelhaltige Haftkleber, Dispersionshaftkleber, Schmelzhaftkleber oder Mischungen daraus umfasst. Die Haftklebstoffe können mittels UV-Bestrahlung und/oder thermisch auf der Trägerschicht verarbeitet werden. Es werden also nicht oder nicht vollständig aushärtende Klebstoffe als Material für die Klebeschicht ausgewählt, welche drucksensitiv sind, jedoch keine chemische Bindung mit den angrenzenden Schichten eingehen.

Weiterhin kann die Releaseschicht des Vorläuferverbundmaterials ein Material aufweisen, das aus einer Gruppe ausgewählt ist, die Polyamid, Polystyrol, thermoplastische Elastomere, Polystyrolcopolymere, Polyester, Polyestercopolymere, Polyolefine und Kombinationen daraus sowie Kombinationen der genannten Materialien einzeln oder Kombinationen der genannten Materialien mit Haftvermittlern umfasst. Beispiele für thermoplastische Elastomere sind TPE-E (thermoplastische Polyestereleastomere bzw. thermoplastische Copolyester, zum Beispiel Hytrel (DuPont) oder Riteflex (Ticona)), TPE-U (thermoplastische Elastomere auf Urethanbasis, beispielsweise Desmopan, Texin, Utechllan (Bayer)), TPE-V (vernetzte thermoplastische Elastomere auf Olefinbasis, vorwiegend PP/EPDM, beispielsweise Sarlink (DSM), Forprene (SoFter)), TPE-O (thermoplastische Elastomere auf Olefinbasis, vorwiegend PP/EPDM, beispielsweise Santoprene (AES/Monsanto)), TPE-S (Styrol-Blockcopolymere wie SBS, SEBS, SEPS, SEEPS und MBS, beispielsweise Styroflex (BASF), Septon (Kuraray) oder Thermolast (Kraiburg TPE)) und TPE-A (thermoplastische Copolyamide, beispielsweise PEBAX (Arkema)). Beispiele für Polyolefine sind Polyethylen oder Polypropylen. Die Releaseschicht kann sich auch aus erster, zweiter und dritter Releaseschicht zusammensetzen, wobei die erste Releaseschicht ein Material aufweist, welches ausgewählt ist aus Polyethylen, Polypropylen, Polyamid, thermoplastischen Elastomeren, Polystyrol, Polystyrolcopolymeren, Polyester, Polyestercopolymeren und Kombinationen daraus, die zweite Releaseschicht ausgewählt ist aus polymeren Haftvermittlern und die dritte Releaseschicht Polyolefine wie beispielsweise Polyethylen oder Polypropylen aufweisen kann. Eine solche Kombination an Releaseschichten kann die Kosten für die Releaseschichten reduzieren, die Steifheit bzw. Weichheit modifizieren und die Stanzbarkeit der Schicht verbessern, was für die Herstellung eines Produkts, in dem ein Verbundmaterial, das aus dem Vorläuferverbundmaterial hergestellt ist, eingesetzt wird, relevant sein kann.

Weiterhin kann das Vorläuferverbundmaterial eine Trägerschicht aufweisen, welche ein Material aufweist, das aus einer Gruppe ausgewählt ist, die Polyolefine, Polyolefincopolymere, thermoplastische Elastomere und Kombinationen daraus umfasst. Beispiele für Polyolefine sind Polyethylen und Polypropylen, Beispiele für thermoplastische Elastomere sind TPE-E, TPE-U, TPE-V, TPE-O, TPE-S und TPE-A. Der Einsatz von thermoplastischen Elastomeren wie TPE-U und TPE-E sind insbesondere von Vorteil, wenn aus dem Vorläuferverbundmaterial ein Verbundmaterial hergestellt werden soll, welches eine atmungsaktive Trägerschicht aufweist. Solche Trägerschichten sind beispielsweise bei der Anwendung des Verbundmaterials in einem Hygienelaminat vorteilhaft.

Die Trägerschicht kann sich aus beispielsweise zwei Schichten zusammensetzen, wobei die erste Trägerschicht ein Material aufweisen kann, welches ausgewählt ist aus Polyethylen, Polypropylen, Polypropylencopolymeren, thermoplastischen Elastomeren sowie Kombinationen daraus. Die zweite Trägerschicht kann ein Material aufweisen, das ausgewählt ist aus einer Gruppe, die Polyethylen, Polypropylen, Polypropylencopolymere, thermoplastische Elastomere sowie Mischungen daraus umfasst.

Bei der Auswahl der Materialien für die Trägerschicht und die Releaseschicht ist darauf zu achten, dass zwischen den gewählten Materialien keine Grenzphasendiffusion auftritt, die Materialien somit gut zu delaminieren sind. Dazu können zumindest 50% der Materialien der Trägerschicht unterschiedlich von den Materialien der Releaseschicht sein bzw. zumindest 50% der Materialien der Releaseschicht unterschiedlich von den Materialien der Trägerschicht sein. Beispielsweise können die unterschiedlichen Materialien der Trägerschicht und der Releaseschicht ein Polyolefin, beispielsweise Polyethylen, und Polystyrol, ein Polyolefin, beispielsweise Polyethylen, und ein thermoplastisches Elastomer wie TPE-U, ein Polyolefin und ein Polyamid, ein Polyester und ein Polyolefin, oder ein Polyethylen niedriger Dichte und Polypropylen Homopolymer ausgewählt werden.

Weiterhin kann die Trennschicht des Vorläuferverbundmaterials ein Material aufweisen, das aus einer Gruppe ausgewählt ist, die Silikon, ausgehärtetes Polysiloxan und thermoplastisches Silikonelastomer umfasst. Diese Verbindungen gehen keine Verbindung mit der Klebeschicht ein, sodass die Klebeschicht geschützt wird, die Trennschicht jedoch inert bleibt. Damit kann die Trennschicht beispielsweise in einem späteren Produkt, in dem ein Verbundmaterial, das aus dem Vorläuferverbundmaterial hergestellt ist, von der Klebeschicht abgezogen werden.

Es wird weiterhin ein Verbundmaterial angegeben, das aus einem Vorläufermaterial gemäß den obigen Ausführungen gebildet ist, und das eine Releaseschicht, eine Trennschicht auf der Releaseschicht, eine Klebeschicht auf der Trennschicht und eine Trägerschicht auf der Klebeschicht umfasst.

Die Merkmale, welche in Bezug auf das Vorläuferverbundmaterial genannt wurden, und die die Releaseschicht, die Trennschicht, die Klebeschicht und die Trägerschicht betreffen, gelten für das Verbundmaterial entsprechend. Somit weist das Verbundmaterial eine Anordnung von Schichten auf, in der eine Trägerschicht und/oder eine Releaseschicht vorhanden sind, welche besonders dünn ausgeführt sein können. Im Vergleich zu herkömmlichen Verbundmaterialien kann weiterhin die Releaseschicht nicht aus Papier, sondern aus einer Folie, die beispielsweise aus Polyamid, Polystyrol, thermoplastischen Elastomeren, Polystyrolcopolymeren, Polyester, Polyestercopolymeren, Polyolefinen, Kombinationen daraus sowie Kombinationen dieser Materialien mit Haftvermittlern hergestellt sein. Somit kann das Verbundmaterial beispielsweise in Hygienelaminaten eingesetzt werden und trägt dort zu verbessertem Tragekomfort beziehungsweise verbessertem Handling bei. Weiterhin kann ein solches Verbundmaterial direkt in einem Produkt eingesetzt werden, ohne dass ein separater Auftrag eines Klebstoffes nötig ist, da dieser bereits in der Klebeschicht des Verbundmaterials vorhanden ist.

Es wird weiterhin ein Verfahren zur Herstellung eines Vorläuferverbundmaterials gemäß den obigen Ausführungen angegeben mit den Verfahrensschritten:
A) Bereitstellen einer Schichtenfolge, die eine Klebeschicht, eine Trägerschicht auf der Klebeschicht, eine Releaseschicht auf der Trägerschicht und eine Trennschicht auf der Releaseschicht enthält, und
B) aufeinander Anordnen der von der Schichtenfolge abgewandten Seite der Klebeschicht und der von der Schichtenfolge abgewandten Seite der Trennschicht zumindest in Teilbereichen der Schichtenfolge.

Mit diesem Verfahren kann somit ein Vorläuferverbundmaterial gemäß den obigen Ausführungen hergestellt werden. Die in Bezug auf das Vorläuferverbundmaterial genannten Merkmale bezüglich der Schichtenfolge, der Klebeschicht, der Trägerschicht, der Releaseschicht und der Trennschicht gelten somit für das Verfahren analog. Ein solches Verfahren ermöglicht eine sehr schnelle Herstellung eines Vorläuferverbundmaterials und die Herstellung eines einfach handzuhabenden Vorläuferverbundmaterials, aus dem nur durch Umsortieren der Abfolge der Schichten ein Verbundmaterial hergestellt werden kann.

Der Verfahrensschritt A) kann die Schritte
A1) Koextrudieren zumindest der Trägerschicht und der Releaseschicht, und
A2) Beschichten zumindest der Trägerschicht mit der Klebeschicht, umfassen.

Die Koextrusion im Verfahrensschritt A1) kann mittels Blasfolienextrusion oder Castfolienextrusion erfolgen.

Es kann im Verfahrensschritt A1) die Trägerschicht und die Releaseschicht koextrudiert werden und im Verfahrensschritt A2) die Trägerschicht mit der Klebeschicht und die Releaseschicht mit der Trennschicht beschichtet werden. Im Verfahrensschritt A1) wird während der Extrusion die Dicke der Trägerschicht und der Releaseschicht eingestellt. Alternativ kann im Verfahrensschritt A1) die Trennschicht, die Releaseschicht und die Trägerschicht koextrudiert werden und im Verfahrensschritt A2) die Trägerschicht mit der Klebeschicht beschichtet werden. In diesem Falle wird im Verfahrensschritt A1) die Dicke der Trennschicht, der Trägerschicht und der Releaseschicht eingestellt sowie ein Laminat hergestellt, in dem die Releaseschicht zwischen der Trägerschicht und der Trennschicht angeordnet ist. Die Beschichtung im Verfahrensschritt A2) erfolgt jeweils auf der von der Trägerschicht abgewandten Seite der Klebeschicht und gegebenenfalls auf der von der von der Releaseschicht abgewandten Seite der Trennschicht.

Im Verfahrensschritt A1) kann somit eine sehr geringe Dicke der Trägerschicht und/oder der Releaseschicht sowie gegebenenfalls der Trennschicht eingestellt werden. Durch die nachfolgende Beschichtung und Anordnung der Schichtenfolge können diese dünnen Schichten ohne Zerstörung weiterverarbeitet werden, da sie nicht alleine einem Zug ausgesetzt werden, sondern nur in einem Laminat von Schichten.

Wenn im Verfahrensschritt A1) die Trägerschicht und die Releaseschicht koextrudiert werden, kann im Verfahrensschritt A2) eine Beschichtung der Releaseschicht mit der Trennschicht erfolgen, wobei die Trennschicht als Material Silikon aufweist. Wird die Trennschicht bereits im Verfahrensschritt A1) mit der Trägerschicht und der Releaseschicht koextrudiert, kann als Material für die Trennschicht ausgehärtetes Polysiloxan und thermoplastisches Silikonelastomer ausgewählt werden.

In dem Verfahren werden die koextrudierten Schichten somit beschichtet und unterliegen sodann nicht mehr einzeln dem Zug, welcher im folgenden Weiterverarbeitungsprozess auf die Schichtenfolge einwirkt.

Im Verfahrensschritt B) kann die Schichtenfolge gewickelt werden. Durch eine Wickelung kann die von der Schichtenfolge abgewandte Seite der Klebeschicht und die von der Schichtenfolge abgewandten Seite der Trennschicht zumindest in Teilbereichen aufeinander angeordnet werden. Durch eine solche Anordnung ist die zur Herstellung eines Verbundmaterials aus dem Vorläuferverbundmaterial notwendige Delamination der Trägerschicht und der Releaseschicht besonders einfach realisierbar.

Es wird weiterhin ein Verfahren zur Herstellung des oben beschriebenen Verbundmaterials angegeben, das eine Releaseschicht, eine Trennschicht auf der Releaseschicht, eine Klebeschicht auf der Trennschicht und eine Trägerschicht auf der Klebeschicht umfasst. Das Verfahren umfasst die Verfahrensschritte
C) Herstellen eines Vorläuferverbundmaterials mit einem Verfahren wie oben beschrieben, und
D) Delaminieren der Trägerschicht und der Releaseschicht des Vorläuferverbundmaterials.

Aus dem wie oben beschrieben hergestellten Vorläuferverbundmaterial kann somit besonders einfach ein Verbundmaterial hergestellt werden, in dem die Trägerschicht und die Releaseschicht des Vorläuferverbundmaterials delaminiert werden. Gleichzeitig bleibt die Verbindung zwischen Klebeschicht und Trennschicht erhalten, sodass eine Neuanordnung der Schichtenfolge des Vorläuferverbundmaterials erfolgt und das Verbundmaterial resultiert. Die Herstellung des Verbundmaterials erfolgt somit unter Verwendung des Vorläuferverbundmaterials und Neuanordnung der Schichten des Vorläuferverbundmaterials.

Mit diesem Verfahren wird somit ein Verbundmaterial bereitgestellt, welches als solches für die Herstellung von beispielsweise Damenbinden, Slipeinlagen, Inkontinenzprodukten, Labels und Etiketten verwendet werden kann ohne dass in der Herstellung ein separater Kleberauftrag notwendig ist.

Es wird weiterhin die Verwendung eines Vorläuferverbundmaterials nach den obigen Ausführungen zur Herstellung eines Verbundmaterials gemäß den obigen Ausführungen angegeben. Ein Vorläuferverbundmaterial wie oben beschrieben kann somit eingesetzt werden, um ein Verbundmaterial mit den oben genannten Eigenschaften herzustellen. Dadurch werden insbesondere verbesserte Eigenschaften des Verbundmaterials erzielt.

Weiterhin wird die Verwendung des Verbundmaterials mit den oben genannten Eigenschaften als Label, Etikett oder als Bestandteil in einem Hygienelaminat angegeben. Label, Etikett oder Hygienelaminat können beispielsweise durch die besonders dünnen Release- und/oder Trägerschicht, die in dem Vorläuferverbundmaterial realisiert werden können, vorteilhafte Eigenschaften aufweisen.

Weitere Vorteile, vorteilhafte Ausführungsformen und Weiterbildungen ergeben sich aus den im Folgenden in Verbindung mit den Figuren beschriebenen Ausführungsbeispielen.
- Figuren 1a und 1b: zeigen schematische Seitenansichten eines Vorläuferverbundmaterials gemäß einer Ausführungsform,
- Figur 2: zeigt die schematische Seitenansicht eines Vorläuferverbundmaterials gemäß einer weiteren Ausführungsform,
- Figur 3: zeigt die schematische Seitenansicht eines Verbundmaterials,
- Figuren 4a, 4b und 4c: zeigen schematische Seitenansichten von koextrudierten Schichten des Vorläuferverbundmaterials,
- Figuren 5a, 5b und 5c: zeigen schematische Seitenansichten von Schichtenfolgen des Vorläuferverbundmaterials gemäß verschiedener Ausführungsformen,
- Figuren 6a, 6b und 6c: zeigen schematische Seitenansichten von Vorläuferverbundmaterialien gemäß verschiedener Ausführungsformen,
- Figuren 7a und 7b: zeigen schematische Seitenansichten des Verfahrens zur Herstellung eines Verbundmaterials unter Verwendung des Vorläuferverbundmaterials,
- Figuren 8a und 8b: zeigen die schematische Seitenansicht beziehungsweise schematische Draufsicht eines Hygienelaminats.

In den Ausführungsbeispielen und Figuren können gleiche, gleichartige oder gleich wirkende Elemente jeweils mit denselben Bezugszeichen versehen sein. Die dargestellten Elemente und deren Größenverhältnisse untereinander sind nicht als maßstabsgerecht anzusehen, vielmehr können einzelne Elemente, wie zum Beispiel Schichten und Bereiche, zur besseren Darstellbarkeit und/oder zum besseren Verständnis übertrieben groß dargestellt sein.

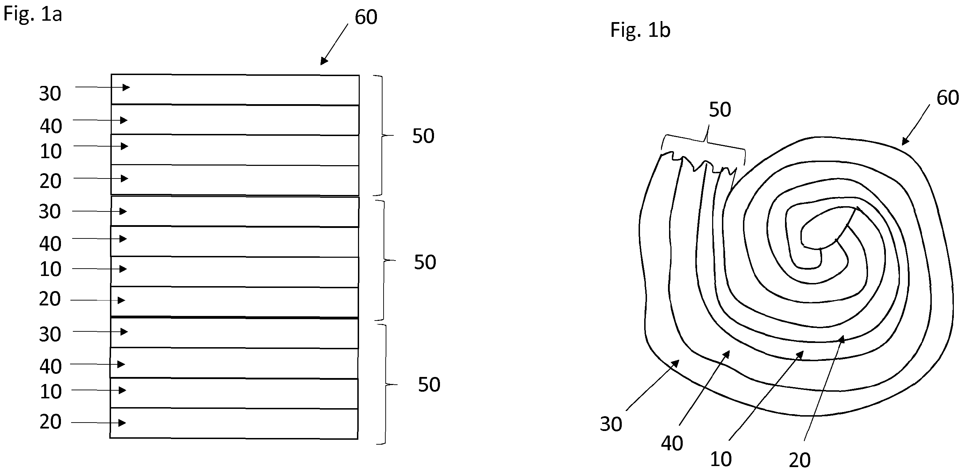

In den Figuren 1a und 1b sind schematische Seitenansichten eines Vorläuferverbundmaterials 60 gezeigt. Dabei handelt es sich jeweils um Ausschnitte des Vorläuferverbundmaterials 60. Figur 1b zeigt die Schichtenfolge 50 des Vorläuferverbundmaterials 60, welche gewickelt vorliegt. Figur 1a zeigt ebenfalls die Schichtenfolge 50, die gewickelt vorliegt, jedoch als Querschnitt durch die Wickelung, sodass mehrere Schichtenfolgen 50 übereinanderliegend angeordnet zu sehen sind. Je nach Größe der Wickelung können deutlich mehr Schichtenfolgen 50 übereinander angeordnet in der Wickelung vorliegen.

Eine Schichtenfolge 50 weist dabei eine Klebeschicht 20, eine Trägerschicht 10, eine Releaseschicht 40 und eine Trennschicht 30 auf. Diese sind übereinander so angeordnet, dass sie gemeinsame Grenzflächen miteinander aufweisen. Durch die Anordnung übereinander weisen zusätzlich die Trennschicht 30 und die Klebeschicht 20 zumindest in Teilbereichen der Schichtenfolge eine gemeinsame Grenzfläche miteinander auf.

Dabei sind die Materialien der Klebeschicht 20 so gewählt, dass die Klebekraft zwischen Klebeschicht 20 und Trennschicht 30 größer ist als die Delaminationskraft zwischen der Trägerschicht und der Releaseschicht 40. Das Material der Klebeschicht 20 ist ein Haftklebstoff, der nach dem Auftragen auf die Trägerschicht dauerklebrig und/oder hochviskos bleibt. Das Material der Trennschicht 30 kann Silikon, ausgehärtetes Polysiloxan oder ein thermoplastisches Silikonelastomer sein. Die Trägerschicht 10 kann Polyethylen, Polypropylen, Polypropylencopolymere oder thermoplastische Elastomere umfassen. Die Releaseschicht 40 weist als Material beispielsweise Polyethylen, Polypropylen, Polyamid, thermoplastische Elastomere, Polystyrol, Polystyrolcopolymere, Polyester und/oder Polyestercopolymere auf.

Die Dicke der Trägerschicht 10 beträgt dabei weniger als 20 µm, bevorzugt weniger als 15 µm, besonders bevorzugt weniger 10 µm, ganz besonders bevorzugt weniger als 5 µm. Die Dicke der Releaseschicht 40 beträgt weniger als 40 µm, bevorzugt weniger als 30 µm, besonders bevorzugt weniger als 20 µm, insbesondere weniger als 15 µm.

Beispielsweise ist die Klebeschicht 20 aus dem Haftklebstoff Solucryl 147 der Firma Henkel (ein Acrylcopolymer) gebildet, die Trägerschicht 10 aus dem auf einem Polyethercopolymer basierenden TPE-U Pearlthane Clear 15N85 der Firma Merquinsa, die Releaseschicht 40 aus dem Polyethylen niedriger Dichte Lupolen 2420 F der Firma LyondellBasell und die Trennschicht 30 aus einem Silikon auf Basis Tego RC 902 (98%) und Tego Photoinitiator A 18 (2%) der Firma EVONIK.

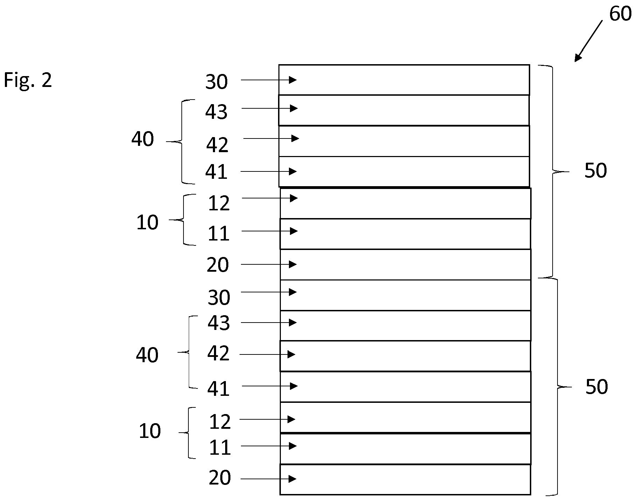

Figur 2 zeigt eine weitere Ausführungsform des Vorläuferverbundmaterials 60 in schematischer Seitenansicht. Auch diese Figur ist wieder als Ausschnitt eines Vorläuferverbundmaterials 60 zu verstehen, in dem nur zwei Schichtenfolgen 50 übereinander angeordnet zu sehen sind. Je nach Anordnung des Vorläuferverbundmaterials beziehungsweise Dicke der Wickelung des Vorläuferverbundmaterials 60 können auch mehrere Schichtenfolgen 50 übereinander angeordnet sein.

In diesem Beispiel enthält die Trägerschicht 10 jeweils zwei Teilschichten, eine erste Trägerschicht 11 und eine zweite Trägerschicht 12. Die erste Trägerschicht 11 kann dabei ein Polymer wie Polyethylen, Polypropylen, Polypropylencopolymere, thermoplastische Elastomere oder Kombinationen daraus umfassen. Die zweite Trägerschicht 12 kann ein Polyethylen, Polypropylen, Polypropylencopolymere sowie thermoplastische Elastomere und Kombinationen daraus umfassen. Die Kombination mehrerer Trägerschichten dient zur Einstellung der Weichheit der Trägerschicht. Dies kann beispielsweise relevant sein, wenn die Trägerschicht in einem Hygienelaminat die Funktion einer Wäscheschutzfolie hat, welche bei Verwendung weich sein muss. Wird als Material der Trägerschichten ein thermoplastisches Elastomer, beispielsweise TPE-E oder TPE-U verwendet, ist die Trägerschicht zudem atmungsaktiv.

Weiterhin ist die Releaseschicht 40 in drei Teilschichten aufgeteilt und enthält die erste Releaseschicht 41, die zweite Releaseschicht 42 sowie die dritte Releaseschicht 43. Die erste Releaseschicht 41 weist dabei beispielsweise ein Polymer auf, das ausgewählt ist aus Polyethylen, Polypropylen, Polyamid, thermoplastische Elastomere, Polystyrol, Polystyrolcopolymere, Polyester und Polyestercopolymere. Die zweite Releaseschicht 42 enthält ein Polymer, das als Haftvermittler dient. Die dritte Releaseschicht 43 kann wiederum beispielsweise Polymere wie Polyethylen oder Polyolefine enthalten. Die Aufteilung der Releaseschicht 40 in drei Teilschichten dient zur Modifikation der Steifheit bzw. Weichheit der Releaseschicht 40. Zudem können, wenn Polyamid als Material der Releaseschicht 40 verwendet wird, die Kosten der Releaseschicht gesenkt werden, indem das teure Polyamid durch die Abfolge Polyolefin/Haftvermittler/Polyamid ersetzt wird. Die Dicken der Trägerschicht 10 und der Releaseschicht 40 entsprechen den Dicken wie in Bezug auf Figur 1a und 1b genannt.

Die Trennschicht 30 dient bei Verwendung des Vorläuferverbundmaterials bzw. bei Verwendung des aus dem Vorläuferverbundmaterials hergestellten Verbundmateril als Schutzbeschichtung der Klebeschicht 20 und zum einfachen Abziehen. Die Klebeschicht 20 kann somit in der Anwendung auf eine Applikationsfläche, beispielsweise auf Textilien, geklebt werden.

In einem beispielhaften Aufbau ist die Klebeschicht 20 aus dem Haftklebstoff Solucryl 147 der Firma Henkel gebildet, die Trägerschicht 10 aus dem Polyethylen hoher Dichte Hostalen GF 9045 F der Firma LyondellBasell, die Trennschicht 30 aus einem Silikon auf Basis Tego RC 902 (98%) und Tego Photoinitiator A 18 (2%) der Firma EVONIK und die Releaseschicht 40 aus drei Teilschichten, wobei die erste Releaseschicht 41 aus dem Polyamid Durethan C 38 F der Firma Lanxess in einer Dicke von 1 µm bis 10 µm gebildet ist, die zweite Releaseschicht 42 aus dem haftvermittlenden auf Homo-Polypropylen basierenden Polymer Admer QB 520 E der Firma Mitsui Chemicals in einer Dicke von 1 µm bis 10 µm und die dritte Releaseschicht 43 aus einem Polyolefin bzw. Polyethylen hoher Dichte wie Hostalen GF 9045 F der Firma LyondellBasell in einer Dicke von 1 µm bis 30 µm.

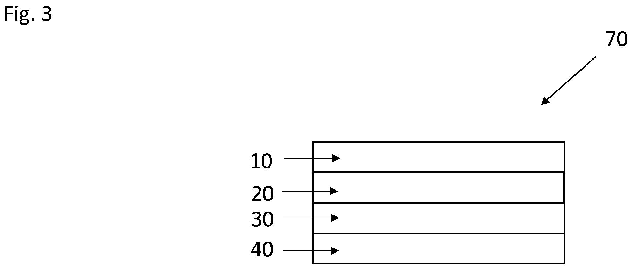

Figur 3 zeigt die schematische Seitenansicht eines Verbundmaterials 70. Dieses weist eine Releaseschicht 40, eine Trennschicht 30 auf der Releaseschicht 40, eine Klebeschicht 20 auf der Trennschicht sowie eine Trägerschicht 10 auf der Klebeschicht 20 auf. Ein solches Verbundmaterial 70 ist aus dem Vorläuferverbundmaterial 60 hergestellt, sodass die Materialien und Schichtdicken der in Bezug auf das Vorläuferverbundmaterial genannten Schichten auch für die Schichten des Verbundmaterials 70 gelten. Somit sind vor allem die Trägerschicht 10 und/oder die Releaseschicht 40 besonders dünn ausgeformt, was das Verbundmaterial 70 geeignet macht, es beispielsweise in einem Hygienelaminat oder als Label einzusetzen. Insbesondere der Einsatz in einem Hygienelaminat führt zu einem verbesserten Tragekomfort aufgrund der dünnen Schichten 10 und 40.

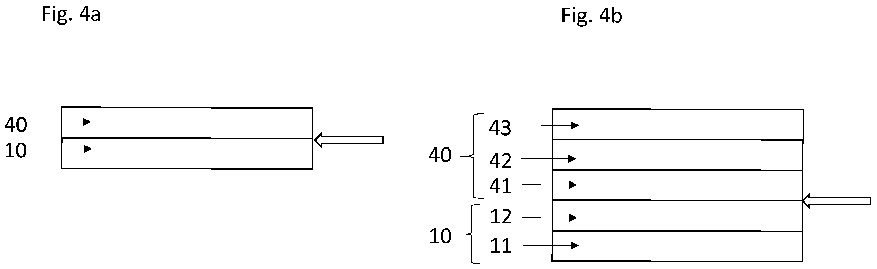

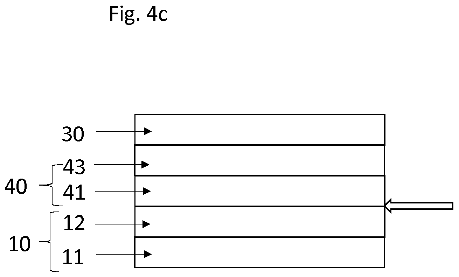

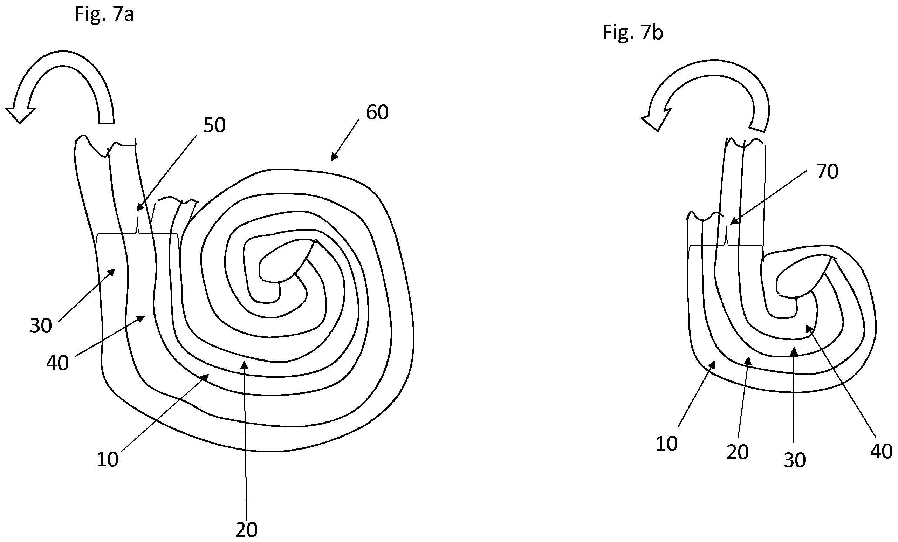

Die folgenden Figuren 4 bis 7 erläutern die Verfahren zur Herstellung des Vorläuferverbundmaterials 60 sowie des Verbundmaterials 70 aus dem Vorläuferverbundmaterial 60. Dabei beziehen sich die Figuren 4 bis 6 auf die Herstellung des Vorläuferverbundmaterials 60, die Figur 7 auf die Herstellung des Verbundmaterials 70. In den Figuren 4 bis 6 werden jeweils drei Ausführungsbeispiele eines Vorläuferverbundmaterials 60 gezeigt beziehungsweise die Schritte zu ihrer Herstellung. Figuren 4a, 5a und 6a beziehen sich auf ein erstes Ausführungsbeispiel, Figuren 4b, 5b und 6b beziehen sich auf ein zweites Ausführungsbeispiel und die Figuren 4c, 5c und 6c beziehen sich auf drittes Ausführungsbeispiel.

Die Figur 4 zeigt schematische Seitenansichten von koextrudierten Schichten des Vorläuferverbundmaterials 60 nach dem Verfahrensschritt A1). Dabei zeigt Figur 4a ein erstes Ausführungsbeispiel, in dem eine Trägerschicht 10 und eine Releaseschicht 40 koextrudiert sind, Figur 4b zeigt ein zweites Ausführungsbeispiel, in dem die Trägerschicht 10 sich aus zwei Teilschichten zusammensetzt und die Releaseschicht 40 sich aus drei Teilschichten zusammensetzt, sodass insgesamt fünf Schichten nach dem Verfahrensschritt A1) koextrudiert vorliegen. Die jeweiligen Materialien der einzelnen Schichten entsprechen denjenigen, wie bezüglich Figuren 1 und 2 genannt. Figur 4c zeigt ein drittes Ausführungsbeispiel, in dem eine erste Trägerschicht 11 und eine zweite Trägerschicht 12 als Trägerschicht 10, eine erste Releaseschicht 41 und eine dritte Releaseschicht 43 als Releaseschicht 40 sowie eine Trennschicht 30 zusammen koextrudiert wurden. In diesem Fall enthält die Trennschicht 30 ein Material, das ausgewählt ist aus einem ausgehärteten Polysiloxan und einem thermoplastischen Silikonelastomer. Solche Materialien können koextrudiert werden und daher ebenfalls besonders dünn ausgeführt sein.

In allen Figuren 4a, 4b und 4c zeigen schematische Pfeile, wo später zur Herstellung eines Verbundmaterials die kontrollierte Delamination stattfindet, nämlich jeweils zwischen der Trägerschicht 10 und der Releaseschicht 40, beziehungsweise der zweiten Trägerschicht 12 und der ersten Releaseschicht 41. Die Delamination wird dadurch ermöglicht, dass zwischen der Klebeschicht 20 und der Trennschicht 30 eine Klebekraft vorhanden ist, die größer ist als die Delaminationskraft zwischen der Trägerschicht 10 und der Releaseschicht 40.

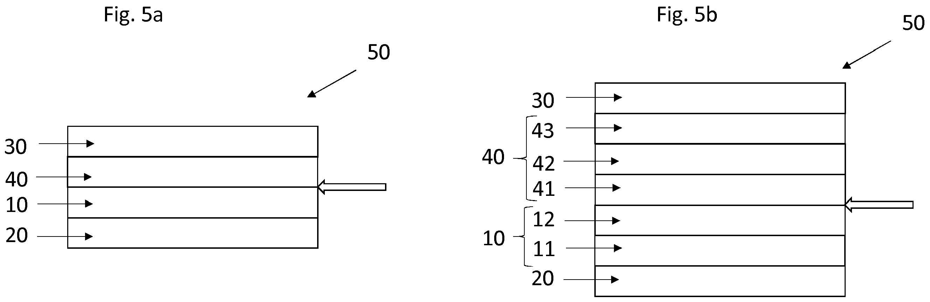

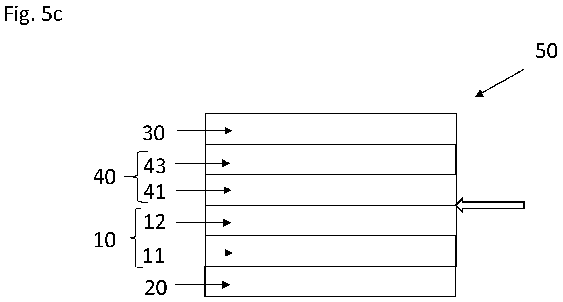

Die Figur 5 zeigt die schematische Seitenansicht von Schichtenfolgen verschiedener Ausführungsformen des Vorläuferverbundmaterials 60. In dem Verfahren zur Herstellung des Vorläuferverbundmaterials 60 handelt es sich dabei um das Zwischenprodukt, das nach dem Verfahrensschritt A) erhalten wird.

Nach der in Figur 4 gezeigten Koextrusion der Trägerschicht 10 und der Releaseschicht 40 , während der auch deren jeweilige Dicke durch Zug eingestellt wird, wird im Verfahrensschritt A2) zumindest die Trägerschicht 10 mit einer Klebeschicht 20 beschichtet. In dem Ausführungsbeispiel gemäß Figur 5a wird die Trägerschicht 10 mit der Klebeschicht 20 und die Releaseschicht 40 mit der Trennschicht 30 beschichtet. In diesem Fall wird als Trennschicht 30 eine Silikonschicht eingesetzt. In dem Ausführungsbeispiel gemäß Figur 5b wird die erste Trägerschicht 11 mit der Klebeschicht 20 beschichtet und die dritte Releaseschicht 43 mit einer Trennschicht 30 ebenfalls beschichtet. Auch in diesem Fall handelt es sich bei der Trennschicht 30 um eine Silikonschicht. In dem Ausführungsbeispiel gemäß Figur 5c wurde die Trennschicht 30 bereits mit der Releaseschicht 40 und der Trägerschicht 10 koextrudiert, sodass nur eine Beschichtung der ersten Trägerschicht 11 mit der Klebeschicht 20 in dem Verfahrensschritt A2) stattgefunden hat. Durch die Beschichtungen werden jeweils Schichtenfolgen 50 erhalten, die jetzt auch bei geringer Dicke von Trägerschicht 10 und/oder Releaseschicht 40 weiterverarbeitet werden können, ohne dass eine Zerstörung der dünnen Schichten auftritt. In den Figuren 5a bis 5c zeigen jeweils schematische Pfeile wieder, wo bei Weiterverarbeitung des Vorläuferverbundmaterials zu einem Verbundmaterial die kontrollierte Delamination der Schichten auftritt.

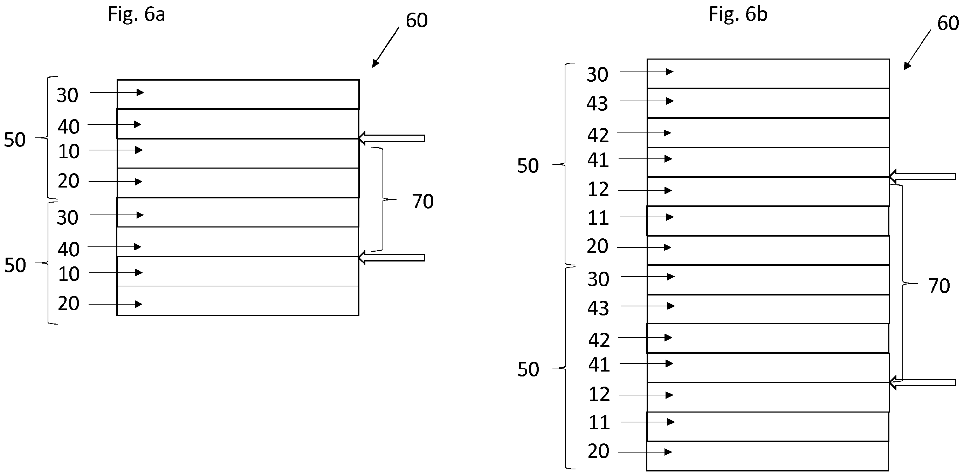

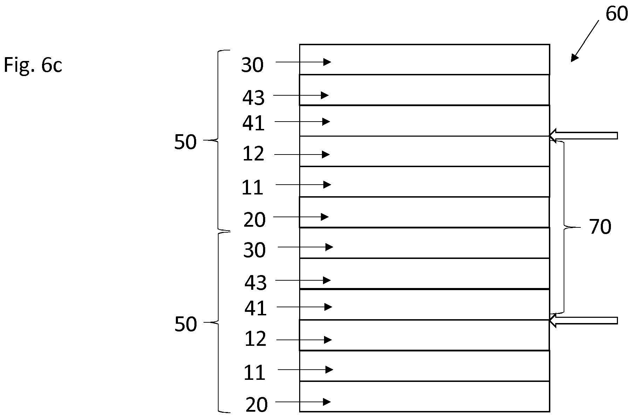

Die Figur 6 zeigt jeweils schematische Seitenansichten des fertiggestellten Vorläuferverbundmaterials 60 der verschiedenen Ausführungsformen, nach dem Verfahrensschritt B), also nach dem Anordnen der Schichtenfolge 50, sodass die Klebeschicht 20 mit ihrer von der Schichtenfolge abgewandten Seite zumindest in Teilbereichen auf der von der Schichtenfolge abgewandte Seite der Trennschicht 30 angeordnet ist. Die schematischen Seitenansichten der Figuren 6a bis 6c sind wieder jeweils als Ausschnitte zu verstehen, da je nach Länge und Anordnung beziehungsweise Wickelung der Schichtenfolge, wesentlich mehr Schichtenfolgen 50 übereinander angeordnet vorliegen können. Die einzelnen Schichtenfolgen 50 der Figuren 6a und 6b wurden oben bereits bezüglich der Figuren 1a und 2 erläutert. Bei den Trennschichten 30 handelt es sich hierbei jeweils um Silikonschichten. Die Schichtenfolge 50, wie sie in Figur 6c gezeigt ist, enthält Trennschichten 30, welche als Material ausgehärtetes Polysiloxan oder thermoplastische Silikonelastomere enthält, weiterhin enthält die Releaseschicht 40 zwei Teilschichten, nämlich die erste Releaseschicht 41 und die dritte Releaseschicht 43.

Auch in den Figuren 6a bis 6c zeigen die schematischen Pfeile wieder, an welcher Stelle eine kontrollierte Delamination bei Auflösung der Anordnung der Schichtenfolgen 50, also beispielsweise bei Abwicklung der Schichtenfolge 50, stattfindet. In den Figuren ist jeweils angedeutet, an welcher Stelle ein Verbundmaterial 70 bei der Umsortierung beziehungsweise der Delamination des Vorläuferverbundmaterials 60 resultieren wird.

Die Figuren 7a und 7b zeigen das Verfahren zur Herstellung des Verbundmaterials 70 aus dem Vorläuferverbundmaterial 60 anhand einer schematischen Seitenansicht eines gewickelten Vorläuferverbundmaterials 60. Diese Figuren sind wieder als Ausschnitt zu verstehen. Figuren 7a und 7b zeigen das Verfahren anhand eines Vorläuferverbundmaterials 60, welches eine Trennschicht 30, in diesem Fall eine Silikonschicht, eine Releaseschicht 40, eine Trägerschicht 10 sowie eine Klebeschicht 20 umfasst. Analoge Verfahren wären auch anwendbar auf Schichtenfolgen 50 des Vorläuferverbundmaterials 60, welche als Trägerschicht 10 und als Releaseschicht 40 jeweils mehrere Teilschichten aufweisen. Bei der kontrollierten Delamination des Vorläuferverbundmaterials 60 wird die Kombination aus Trennschicht 30 und Releaseschicht 40 zunächst alleine abgewickelt (Figur 7a), was besonders einfach möglich ist, da die Delaminationskraft zwischen der Trägerschicht 10 und der Releaseschicht 40 geringer ist als die Klebekraft zwischen der Klebeschicht 20 und der Trennschicht 30, auf der die Klebeschicht 20 in Teilbereichen der Schichtenfolge 50 angeordnet ist. Sobald einmal die halbe Schichtenfolge aus Trennschicht 30 und Releaseschicht 40 abgewickelt ist und dadurch der Teilbereich der gewickelten Schichtenfolge erreicht wird, in dem die Trennschicht 30 auf der Klebeschicht 20 angeordnet ist, wird das Verbundmaterial 70, in dem Trägerschicht 10, Klebeschicht 20, Trennschicht 30 und Releaseschicht 40 übereinander angeordnet sind, abgewickelt, da auch hier wieder die Delaminationskraft zwischen der Releaseschicht 40 und der Trägerschicht 10 geringer ist als die Klebekraft zwischen der Klebeschicht 20 und der Trennschicht 30. Somit kann in besonders einfacher Weise das Verbundmaterial 70 aus dem Vorläuferverbundmaterial 60 gewonnen werden (Figur 7b).

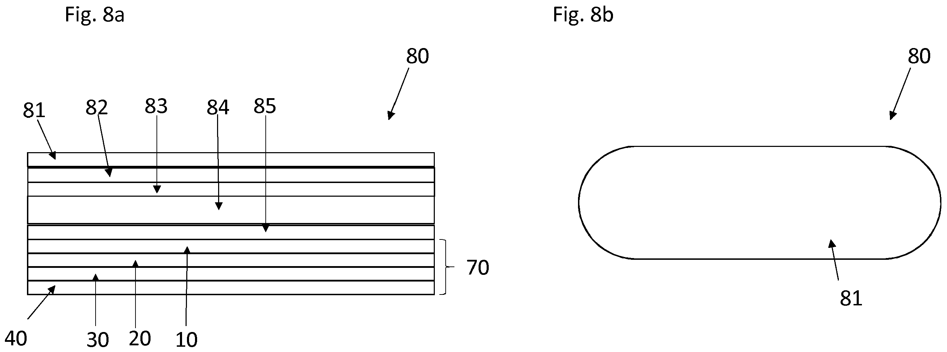

Figur 8 zeigt die schematische Seitenansicht (Figur 8a) beziehungsweise eine schematische Draufsicht (Figur 8b) eines Hygienelaminats, in welchem ein Verbundmaterial 70, das aus dem Vorläuferverbundmaterial 60 hergestellt ist, Anwendung findet.

In Figur 8a ist das Verbundmaterial 70 bestehend aus Releaseschicht 40, Trennschicht 30 auf der Releaseschicht 40, Klebeschicht 20 auf der Trennschicht 30 sowie eine Trägerschicht 10 auf der Klebeschicht 20 so angeordnet, dass auf der Trägerschicht 10 weitere Schichten des Hygienelaminats angeordnet werden. Diese umfassen eine Verklebung 85, einen Saugkern 84 auf der Verklebung 85, eine Verteilerauflage 83 auf dem Saugkern 84, eine Verklebung 82 auf der Verteilerauflage 83 und eine Vliesabdeckung 81 auf der Verklebung 82. Die Trägerschicht 10 kann in diesem Fall als Wäscheschutzfolie ausgebildet sein. Wird das Hygienelaminat von einem Verbraucher verwendet, kann die Releasefolie 40 zusammen mit der Trennschicht 30 abgezogen werden, und das Hygienelaminat mit der Klebeschicht 20 in der Wäsche wieder ablösbar befestigt werden.

Figur 8b zeigt eine Draufsicht auf das Hygienelaminat 80, in welcher man nur die Vliesabdeckung 81 sieht.

In einer Anwendung des Verbundmaterials wie in Figur 8 gezeigt ist es vorteilhaft, wenn die Trägerschicht 10 atmungsaktiv ausgeformt ist, was dadurch erreicht werden kann, wenn TPE-U oder TPE-E als Material der Trägerschicht 10 eingesetzt wird.

Die Erfindung ist nicht durch die Beschreibung anhand der Ausführungsbeispiele beschränkt. Vielmehr umfasst die Erfindung jedes neue Merkmal sowie jede Kombination von Merkmalen, was insbesondere jede Kombination von Merkmalen in den Patentansprüchen beinhaltet, auch wenn dieses Merkmal oder diese Kombination selbst nicht explizit in den Patentansprüchen oder Ausführungsbeispielen angegeben ist.

### Bezugszeichenliste

- 10: Trägerschicht
- 11: erste Trägerschicht
- 12: zweite Trägerschicht
- 20: Klebeschicht
- 30: Trennschicht
- 40: Releaseschicht
- 41: erste Releaseschicht
- 42: zweite Releaseschicht
- 43: dritte Releaseschicht
- 50: Schichtenfolge
- 60: Vorläuferverbundmaterial
- 70: Verbundmaterial
- 80: Hygienelaminat
- 81: Vliesabdeckung
- 82: Verklebung
- 83: Verteilerauflage
- 84: Saugkern
- 85: Verklebung

## Patentansprüche

1. Vorläuferverbundmaterial (60), aufweisend eine Schichtenfolge (50), die eine Klebeschicht (20), eine Trägerschicht (10) auf der Klebeschicht (20), eine Releaseschicht (40) auf der Trägerschicht (10) und eine Trennschicht (30) auf der Releaseschicht (40) enthält, wobei die Schichtenfolge (50) so angeordnet ist, dass die Klebeschicht (20) mit ihrer von der Schichtenfolge (50) abgewandten Seite zumindest in Teilbereichen auf der von der Schichtenfolge (50) abgewandten Seite der Trennschicht (30) angeordnet ist, wobei zwischen der Klebeschicht (20) und der Trennschicht (30) eine Klebekraft vorhanden ist, die größer ist als eine Delaminationskraft zwischen der Trägerschicht (10) und der Releaseschicht (40).

2. Vorläuferverbundmaterial (60) nach dem vorhergehenden Anspruch, wobei die Schichtenfolge (50) gewickelt ist.

3. Vorläuferverbundmaterial (60) nach einem der vorhergehenden Ansprüche, wobei die Releaseschicht (40) eine Dicke aufweist, die kleiner als 40 µm ist und/oder die Trägerschicht (10) eine Dicke aufweist, die kleiner als 20 µm ist.

4. Vorläuferverbundmaterial (60) nach einem der vorhergehenden Ansprüche, wobei die Klebeschicht (20) ein Material aufweist, das aus einer Gruppe ausgewählt ist, die Haftklebstoffe umfasst.

5. Vorläuferverbundmaterial (60) nach einem der vorhergehenden Ansprüche, wobei die Releaseschicht (40) ein Material aufweist, das aus einer Gruppe ausgewählt ist, die Polyamid, Polystyrol, thermoplastische Elastomere, Polystyrolcopolymere, Polyester, Polyestercopolymere, Polyolefine, Kombinationen daraus und Kombinationen aus Polyamid, Polystyrol, thermoplastischen Elastomeren, Polystyrolcopolymeren, Polyestern, Polyestercopolymeren, und/oder Polyolefinen mit Haftvermittlern umfasst.

6. Vorläuferverbundmaterial (60) nach einem der vorhergehenden Ansprüche, wobei die Trägerschicht (10) ein Material aufweist, das aus einer Gruppe ausgewählt ist, die Polyolefine, Polyolefincopolymere, thermoplastische Elastomereund Kombinationen daraus umfasst.

7. Vorläuferverbundmaterial (60) nach einem der vorhergehenden Ansprüche, wobei die Trennschicht (30) ein Material aufweist, das aus einer Gruppe ausgewählt ist, die Silikon, ausgehärtetes Polysiloxan und thermoplastisches Silikonelastomer umfasst.

8. Verbundmaterial (70), das aus einem Vorläuferverbundmaterial (60) gemäß den Ansprüchen 1 bis 7 gebildet ist, umfassend eine Releaseschicht (40), eine Trennschicht (30) auf der Releaseschicht (40), eine Klebeschicht (20) auf der Trennschicht (30) und eine Trägerschicht (10) auf der Klebeschicht (20).

9. Verfahren zur Herstellung eines Vorläuferverbundmaterials (60) nach einem der Ansprüche 1 bis 7, mit den Verfahrensschritten
A) Bereitstellen einer Schichtenfolge (50), die eine Klebeschicht (20), eine Trägerschicht (10) auf der Klebeschicht (20), eine Releaseschicht (40) auf der Trägerschicht (10) und eine Trennschicht (30) auf der Releaseschicht (40) enthält,
B) Aufeinander Anordnen der von der Schichtenfolge (50) abgewandten Seite der Klebeschicht (20) und der von der Schichtenfolge abgewandten Seite der Trennschicht (30) zumindest in Teilbereichen der Schichtenfolge (50).

10. Verfahren nach dem vorhergehenden Anspruch, wobei der Verfahrensschritt A) die Schritte
A1) Koextrudieren zumindest der Trägerschicht (10) und der Releaseschicht (40), und
A2) Beschichten zumindest der Trägerschicht (10) mit der Klebeschicht (20),
umfasst.

11. Verfahren nach dem vorhergehenden Anspruch, wobei im Verfahrensschritt A1) die Trägerschicht (10) und die Releaseschicht (40) koextrudiert werden und im Verfahrensschritt A2) die Trägerschicht (10) mit der Klebeschicht (20) und die Releaseschicht (40) mit der Trennschicht (20) beschichtet werden, oder wobei im Verfahrensschritt A1) die Trennschicht (30), die Releaseschicht (40) und die Trägerschicht (10) koextrudiert werden und im Verfahrensschritt A2) die Trägerschicht (10) mit der Klebeschicht (20) beschichtet wird.

12. Verfahren nach einem der Ansprüche 9 bis 11, wobei im Verfahrensschritt B) die Schichtenfolge (50) gewickelt wird.

13. Verfahren zur Herstellung eines Verbundmaterials (70) nach Anspruch 8 mit den Verfahrensschritten
C) Herstellen eines Vorläuferverbundmaterials (60) mit einem Verfahren gemäß den Ansprüchen 9 bis 12,
D) Delaminieren der Trägerschicht (10) und der Releaseschicht (40) des Vorläuferverbundmaterials (60).

14. Verwendung eines Vorläuferverbundmaterials (60) nach einem der Ansprüche 1 bis 7 zur Herstellung eines Verbundmaterials (70) gemäß Anspruch 8.

15. Verwendung eines Verbundmaterials (70) nach Anspruch 8 als Label, Etikett oder als Bestandteil in einem Hygienelaminat.

## Claims

1. Precursor composite material (60), comprising a layer sequence (50) that contains an adhesive layer (20), a carrier layer (10) on the adhesive layer (20), a release layer (40) on the carrier layer (10) and a parting layer (30) on the release layer (40), wherein the layer sequence (50) is arranged in such a way that the side of the adhesive layer (20) facing away from the layer sequence (50) is arranged at least in sub-regions on the side of the parting layer (30) facing away from the layer sequence (50), wherein an adhesive force is present between the adhesive layer (20) and the parting layer (30) which is greater than a delamination force between the carrier layer (10) and the release layer (40).

2. Precursor composite material (60) according to the preceding claim, wherein the layer sequence (50) is wound.

3. Precursor composite material (60) according to one of the preceding claims, wherein the release layer (40) has a thickness of less than 40 µm and/or the carrier layer (10) has a thickness of less than 20 µm.

4. Precursor composite material (60) according to one of the preceding claims, wherein the adhesive layer (20) comprises a material selected from a group comprising pressure-sensitive adhesives.

5. Precursor composite material (60) according to one of the preceding claims, wherein the release layer (40) has a material selected from a group comprising polyamide, polystyrene, thermoplastic elastomers, polystyrene copolymers, polyester, polyester copolymers, polyolefins, combinations thereof and combinations of polyamide, polystyrene, thermoplastic elastomers, polystyrene copolymers, polyesters, polyester copolymers and/or polyolefins with adhesion promotors.

6. Precursor composite material (60) according to one of the preceding claims, wherein the carrier layer (10) comprises a material selected from a group comprising polyolefins, polyolefin copolymers, thermoplastic elastomers and combinations thereof.

7. Precursor composite material (60) according to one of the preceding claims, wherein the parting layer (30) comprises a material selected from a group comprising silicone, hardened polysiloxane and thermoplastic silicone elastomer.

8. Composite material (70), which is produced from a precursor composite material (60) according to claims 1 to 7, including a release layer (40), a parting layer (30) on the release layer (40), an adhesive layer (20) on the parting layer (30), and a carrier layer (10) on the adhesive layer (20) .

9. Method for producing a precursor composite material (60) according to one of claims 1 to 7, comprising the method steps
A) Providing a layer sequence (50) that contains an adhesive layer (20), a carrier layer (10) on the adhesive layer (20), a release layer (40) on the carrier layer (10) and a parting layer (30) on the release layer (40);
B) Arranging the side of the adhesive layer (20) facing away from the layer sequence (50) and the side of the parting layer (30) facing away from the layer sequence on top of one another at least in sub-regions of the layer sequence (50).

10. Method according to the preceding claim, wherein method step A) includes the steps
A1) Coextruding at least the carrier layer (10) and the release layer (40), and
A2) Coating at least the carrier layer (10) with the adhesive layer (20).

11. Method according to the preceding claim, wherein the carrier layer (10) and the release layer (40) are coextruded in method step A1) and the carrier layer (10) is coated with the adhesive layer (20) and the release layer (40) with the parting layer (20) in method step A2), or wherein the parting layer (30), the release layer (40) and the carrier layer (10) are coextruded in method step A1) and the carrier layer (10) is coated with the adhesive layer (20) in method step A2).

12. Method according to one of claims 9 to 11, wherein the layer sequence (50) is wound in step B).

13. Method for producing a composite material (70) according to claim 8 comprising the method steps of
C) Producing a precursor composite material (60) by providing a layer sequence by a method according to claims 9 to 12,
D) Delaminating the carrier layer (10) and the release layer (40) of the precursor composite material (60).

14. Use of a precursor composite material (60) according to one of claims 1 to 7 for producing a composite material (70) according to claim 8.

15. Use of a composite material (70) according to claim 8 as a label, sticker or a component in a hygienic laminate.

## Revendications

1. Matériau composite précurseur (60) présentant une succession de couches (50) qui comprend une couche adhésive (20), une couche de support (10) sur la couche adhésive (20), une couche antiadhésive (40) sur la couche de support (10) et une couche de séparation (30) sur la couche antiadhésive (40), la succession de couches (50) étant disposée de manière à ce que la couche adhésive (20), avec son côté détourné de la succession de couches (50), au moins dans des zones partielles, soit disposée sur le côté de la couche de séparation (30), détourné de la succession de couches (50), une force adhésive étant présente entre la couche adhésive (20) et la couche de séparation (30), laquelle est supérieure à une force de délamination entre la couche de support (10) et la couche antiadhésive (40).

2. Matériau composite précurseur (60) selon la revendication précédente, la succession de couches (50) étant enroulée.

3. Matériau composite précurseur (60) selon l'une quelconque des revendications précédentes, la couche antiadhésive (40) présentant une épaisseur qui est inférieure à 40 µm et/ou la couche de support (10) présentant une épaisseur qui est inférieure à 20 µm.

4. Matériau composite précurseur (60) selon l'une quelconque des revendications précédentes, la couche adhésive (20) présentant une matériau qui est sélectionnée dans un groupe comprenant des matières adhésives sensibles à pression.

5. Matériau composite précurseur (60) selon l'une quelconque des revendications précédentes, la couche antiadhésive (40) présentant une matière qui est sélectionnée dans un groupe comprenant du polyamide, du polystyrène, des élastomères thermoplastiques, des copolymères de polystyrène, du polyester, des copolymères de polyester, des polyoléfines, des combinaisons de ces matières et des combinaisons constituées de polyamide, de polystyrène, d'élastomères thermoplastiques, de copolymères de polystyrène, de polyesters, de copolymères de polyester et/ou de polyoléfines comprenant des agents adhésifs.

6. Matériau composite précurseur (60) selon l'une quelconque des revendications précédentes, la couche de support (10) présentant une matière qui est sélectionnée dans un groupe comprenant des polyoléfines, des copolymères de polyoléfine, des élastomères thermoplastiques et des combinaisons de ces matières.

7. Matériau composite précurseur (60) selon l'une quelconque des revendications précédentes, la couche de séparation (30) présentant une matière qui est sélectionnée dans un groupe comprenant de la silicone, du polysiloxane durci et du élastomère de silicone thermoplastique.

8. Matériau composite (70) qui est formé à partir d'un matériau composite précurseur (60) selon les revendications 1 à 7, comprenant une couche antiadhésive (40), une couche de séparation (30) sur la couche antiadhésive (40), une couche adhésive (20) sur la couche de séparation (30) et une couche de support (10) sur la couche adhésive (20).

9. Procédé de fabrication d'un matériau composite précurseur (60) selon l'une quelconque des revendications 1 à 7, comprenant les étapes de procédé de
A) la fourniture d'une succession de couches (50) qui comprend une couche adhésive (20), une couche de support (10) sur la couche adhésive (20), une couche antiadhésive (40) sur la couche de support (10) et une couche de séparation (30) sur la couche antiadhésive (40)
B) la disposition de l'une sur l'autre du côté de la couche adhésive (20), détourné de la succession de couches (50), et du côté de la couche de séparation (30), détourné de la succession de couches au moins dans des zones partielles de la succession de couches (50).

10. Procédé selon la revendication précédente, l'étape de procédé A) comprenant les étapes
A1) coextrusion au moins de la couche de support (10) et de la couche antiadhésive (40), et
A2) revêtement au moins de la couche de support (10) par la couche adhésive (20).

11. Procédé selon la revendication précédente, la couche de support (10) et la couche antiadhésive (40) étant coextrudées à l'étape de procédé A1) et, à l'étape de procédé A2), la couche de support (10) étant revêtue de la couche adhésive (20) et la couche antiadhésive(40) étant revêtue de la couche de séparation (20), ou, à l'étape A1), la couche de séparation (30), la couche antiadhésive (40) et la couche de support (10) étant coextrudées et, à l'étape A2), la couche de support (10) étant revêtue de la couche adhésive (20).

12. Procédé selon l'une quelconque des revendications 9 à 11, la succession de couches (50) étant enroulée à l'étape B).

13. Procédé de fabrication d'un matériau composite (70) selon la revendication 8, comprenant les étapes de procédé
C) fabrication d'un matériau composite précurseur (60) à l'aide du procédé selon les revendications 9 à 12,
D) délamination de la couche de support (10) et de la couche antiadhésive (40) du matériau composite précurseur (60).

14. Utilisation d'un matériau composite précurseur (60) selon l'une quelconque des revendications 1 à 7 pour la fabrication d'un matériau composite (70) selon la revendication 8.

15. Utilisation d'un matériau composite (70) selon la revendication 8 en tant que label, étiquette ou en tant que composant dans un stratifié hygiénique.
